# FASCICULE DE BREVET EUROPEEN

(11) **EP 3 749 261 B1**
(45) Date de publication et mention de la délivrance du brevet: **17.11.2021**
(21) Numéro de dépôt: 19705133.7
(22) Date de dépôt: 05.02.2019
(51) Int. Cl.: A61F 9/00

(54) **DISPOSITIF D'ASSISTANCE À L'UTILISATION D'UN DISPOSITIF DE DISTRIBUTION DE PRODUIT LIQUIDE SOUS FORME DE GOUTTES**
VORRICHTUNG ZUR UNTERSTÜTZUNG BEI DER VERWENDUNG EINER VORRICHTUNG ZUR ABGABE VON FLÜSSIGEN PRODUKTEN IN FORM VON TROPFEN
DEVICE FOR ASSISTING IN THE USE OF A DEVICE FOR DISPENSING LIQUID PRODUCT IN THE FORM OF DROPS

(30) Priorité: 06.02.2018 FR 1850984
(43) Date de publication de la demande: 16.12.2020
(73) Titulaire: NEMERA LA VERPILLIERE, 38290 La Verpillière (FR)
(72) Inventeur: DECOCK, Thierry, 69007 Lyon (FR)
(74) Mandataire: LLR
(86) Numéro de dépôt international: PCT/EP2019/052776
(87) Numéro de publication internationale: WO 2019/154806

(56) Documents cités:
- WO-A1-2014/066546
- WO-A1-2016/183394
- US-A- 4 960 407
- US-A1- 2004 176 754

## Description

L'invention concerne un dispositif d'assistance à l'utilisation d'un dispositif de distribution d'un produit liquide sous formes de gouttes.

Quand on distribue un produit liquide sous forme de gouttes, il peut être difficile de tenir et utiliser correctement le dispositif de distribution pour que les gouttes atteignent l'organe cible. Une mauvaise manipulation du dispositif risque de distribuer des gouttes en dehors de l'organe cible, voire de blesser le sujet.

Le document WO2016/183394 décrit un dispositif d'administration de gouttes capable de détecter des couleurs à l'aide de capteurs de couleurs et de les comparer avec une gamme de couleurs prédéterminées pour détecter la présence d'un oeil et un éventuel clin d'oeil. Ce dispositif permet de s'assurer qu'un oeil est positionné en face de l'orifice de distribution au moment de la distribution d'une goutte. Cependant, il ne permet pas de garantir que la goutte atteint effectivement l'oeil car la goutte peut notamment dévier par rapport à l'œil, même si ce dernier est détecté par les capteurs de couleurs. De plus, il peut donner une indication erronée selon laquelle un oeil est présent lorsque la peau ou un objet ayant des couleurs similaires à celles d'un oeil est positionné devant l'orifice de distribution.

Le document WO-A-2014/066546 décrit un dispositif d'assistance à l'utilisation d'un dispositif de distribution d'un produit liquide sous forme de gouttes dans un organe de réception d'un sujet selon le préambule de la revendication 1.

L'invention a notamment pour but de fournir un dispositif d'assistance à l'utilisation d'un dispositif de distribution qui facilite sa manipulation et qui assure qu'une goutte atteint l'organe cible.

À cet effet, l'invention a notamment pour objet un dispositif d'assistance à l'utilisation d'un dispositif de distribution d'un produit liquide sous forme de gouttes dans un organe de réception d'un sujet, le dispositif de distribution comprenant un réservoir, le dispositif d'assistance comprenant :
- des moyens de solidarisation au dispositif de distribution,
- une surface d'appui contre la peau du sujet, disposée au voisinage d'un orifice de distribution du produit liquide,
- des moyens de détection d'un contact entre la surface d'appui et la peau du sujet.

Grâce à la présence de la surface d'appui, on peut immobiliser la position de l'orifice de distribution par rapport à l'organe de réception, et en particulier la distance entre l'orifice de distribution et l'organe de réception. Cela permet de limiter les risques que la goutte soit déviée, en garantissant qu'elle est toujours distribuée à une distance optimale entre l'orifice de distribution et l'organe de réception, et ainsi qu'elle atteint effectivement l'organe de réception. En effet, pour positionner le dispositif de distribution par rapport à l'organe de réception, le sujet plaque la surface d'appui contre la peau entourant l'organe de réception et n'a plus besoin de viser de manière approximative, comme c'est le cas lorsqu'il n'y a pas d'appui, l'organe de réception. Ainsi, la surface d'appui assiste le sujet dans le positionnement et la manipulation du dispositif de distribution puisqu'il suffit au sujet de plaquer la surface d'appui contre la peau de l'organe de réception, ou autour de l'organe de réception, pour distribuer une goutte dans l'organe de réception à une distance adaptée.

En outre, les moyens de détection d'un contact entre la surface d'appui et la peau du sujet permettent d'indiquer au dispositif d'assistance que le dispositif de distribution est a priori correctement positionné, et donc prêt pour distribuer le liquide. Cela permet notamment de garantir que la goutte est effectivement distribuée dans l'organe de réception pour éviter, par exemple, un gaspillage ou une erreur dans le décompte de gouttes à administrer. Quand il détecte le contact, le dispositif d'assistance peut traiter l'information de multiples façons, un exemple étant d'autoriser la distribution ou encore de prévenir le sujet pour que celui-ci déclenche la distribution.

On notera que l'organe de réception peut être un oeil, une oreille, une partie de peau ou tout autre organe de l'être vivant. Le sujet peut être humain ou encore un animal.

Le dispositif d'assistance peut en outre comporter l'une ou plusieurs des caractéristiques suivantes, prises seules ou en combinaison.

― Le dispositif d'assistance comprend des moyens d'écartement configurés pour préserver une distance prédéterminée entre la surface d'appui et l'orifice de distribution des gouttes. La distance entre la surface d'appui et l'orifice de distribution représente la distance que la goutte doit parcourir pour atteindre l'organe de réception. Cette distance est de préférence relativement faible, de l'ordre de quelques centimètres, par exemple de 1 à 3 cm, pour éviter tout risque de déviation de la goutte. Elle permet dans certains cas d'éviter un contact direct entre l'orifice de distribution et l'organe cible pour ne pas blesser le sujet et éventuellement ne pas contaminer l'orifice de distribution. En configurant une distance prédéterminée, l'utilisation du dispositif de distribution devient donc simple et sécurisée, garantissant une distribution de la goutte dans l'organe de réception.

― Le dispositif d'assistance comprend des moyens de détection d'une couleur d'une zone disposée face à la surface d'appui et/ou l'orifice de distribution des gouttes, et éventuellement un système de traitement des informations pour comparer la couleur détectée à au moins une couleur prédéterminée. Ainsi, on peut détecter une couleur caractérisante de l'organe de réception ou encore comparer la couleur détectée à une ou plusieurs couleurs prédéterminées caractérisantes de l'organe de réception. Cela permet de s'assurer que l'orifice de distribution est positionné face à l'organe de réception, ce qui est particulièrement intéressant si l'organe de réception a une couleur distincte de la partie du sujet entourant l'organe de réception. En particulier lorsque l'organe de réception est un oeil, cela permet de vérifier que l'oeil est ouvert, puisque les couleurs potentielles d'un œil ouvert se distinguent par exemple de la couleur d'une paupière. Les moyens de détection de couleurs permettent en outre de valider l'information obtenue par les moyens de détection d'un contact et de favoriser par exemple la distribution de la goutte dans un oeil ouvert. Selon un exemple, les moyens de détection peuvent être configurés pour détecter une ou plusieurs couleurs de la zone disposée face à la surface d'appui et/ou l'orifice de distribution des gouttes, et ensuite la comparer à une gamme de couleurs prédéterminées grâce au système de traitement. Ils peuvent en outre, ou alternativement, fournir un signal positif ou négatif en fonction de la couleur détectée.

― Le dispositif d'assistance comprend des moyens de verrouillage de la distribution de produit aptes à n'autoriser l'activation du dispositif de distribution qu'en fonction du contact détecté et/ou d'une couleur détectée. Avantageusement, les moyens de verrouillage peuvent bloquer la distribution de goutte quand il n'y a pas de contact et/ou de couleur détectée, et attendre que le contact et/ou la couleur soit détecté pour déclencher automatiquement la distribution. Par exemple, quand l'organe de réception est un oeil, les moyens de verrouillage peuvent bloquer la distribution de goutte quand l'oeil est fermé, même si le sujet active la distribution, et attendre que l'oeil s'ouvre pour déclencher la distribution. Les moyens de verrouillage peuvent verrouiller la distribution de produit en fonction de l'information fournie par les moyens de détection d'un contact ou par des moyens de détection de couleurs, ou encore par les deux. On comprend que le cas dans lequel l'activation n'est autorisée que lorsque à la fois un contact et une couleur sont détectés est particulièrement avantageux puisqu'il assure de façon particulièrement fiable que toute goutte distribuée est effectivement reçue dans l'organe de réception. On entend par « activation du dispositif de distribution » le fait qu'un sujet exerce une pression, directement ou à travers le dispositif d'assistance, sur le dispositif de distribution pour distribuer une goutte de liquide.

― Le dispositif d'assistance comprend des moyens optiques destinés à être disposés au voisinage de l'orifice de distribution des gouttes et configurés pour fournir une information sur la distribution d'une goutte du produit liquide par le dispositif de distribution. Plus précisément, les moyens optiques permettent de détecter la présence ou l'absence d'une goutte au voisinage de l'orifice de distribution, donnant une information sur la formation d'une goutte et/ou l'absence d'une goutte, donc une information sur la distribution de la goutte. Ainsi en les combinant avec les moyens de détection d'un contact, on obtient une garantie supplémentaire sur la distribution effective d'une goutte dans l'organe visé. Les moyens optiques détectent de préférence la présence d'une goutte en détectant une perturbation d'un signal optique causée par cette présence.

― Le dispositif d'assistance comprend des moyens de mesure de l'inclinaison configurés pour fournir une information sur l'inclinaison du dispositif de distribution solidarisé au dispositif d'assistance, et un système de traitement des informations sur la distribution d'une goutte et sur l'inclinaison du dispositif de distribution solidarisé au dispositif d'assistance pour fournir une information sur la quantité de produit liquide distribuée. On constate que l'inclinaison du dispositif de distribution lors de la distribution d'une goutte influence le volume de la goutte distribuée. En effet en général, plus l'axe de l'orifice de distribution est orienté perpendiculairement à la surface de l'organe de réception et plus la goutte est grosse. Ainsi, en mesurant l'inclinaison et en fournissant cette information au système de traitement, ce dernier peut estimer le volume de la goutte distribuée et calculer éventuellement le volume restant dans le dispositif de distribution.

- Le dispositif d'assistance comprend des moyens de mesure du poids du dispositif de distribution solidarisé au dispositif d'assistance, configurés pour fournir une information sur la quantité de produit liquide restant dans le dispositif de distribution. En pesant le dispositif de distribution, on obtient le poids du produit liquide restant et connaissant sa densité, on peut obtenir le volume de produit liquide restant qui peut être éventuellement traduit en nombre de gouttes restantes. Connaissant le volume de produit restant par pesée avant et après la distribution d'une goutte, on peut également connaître la quantité de produit distribué. On peut éventuellement croiser la valeur obtenue avec celle obtenue par d'autres moyens, par exemple par mesure de l'inclinaison du dispositif d'assistance, et ainsi effectuer une double validation de la quantité de produit distribuée estimée.

― Le dispositif d'assistance comprend des moyens de réglage de la surface d'appui, de façon à pouvoir faire varier la distance maintenue entre l'orifice de distribution et au moins une partie de la surface d'appui. L'utilisateur peut ainsi adapter la distance de distribution selon ses besoins et son confort, tout en garantissant une distribution correcte de la goutte dans l'organe de réception puisque après réglage, la distance entre la surface d'appui et l'orifice de distribution est maintenue fixe. Les moyens de réglage peuvent comprendre par exemple une vis sans fin ou un ressort combiné à des moyens de verrouillage ou encore un embout remplaçable.

- Les moyens de détection d'un contact comprennent des moyens de détection de lumière configurés pour détecter une variation de l'intensité de la lumière sur une partie de la surface d'appui. Lorsque le sujet appuie la surface d'appui contre la peau, cette dernière étant opaque, bloque au moins partiellement l'arrivée de la lumière sur au moins une partie de la surface d'appui, causant une variation de l'intensité de la lumière sur cette partie de la surface d'appui. Ainsi en détectant cette variation, on détecte le contact entre la peau et la surface d'appui. Pour détecter la variation de l'intensité de la lumière, les moyens de détection de lumière peuvent comprendre des capteurs de lumière disposés à la surface de la surface d'appui, de préférence sous une partie perméable à la lumière de la surface d'appui. Les moyens de détection de lumière peuvent être également disposés dans un tunnel débouchant sur la surface d'appui ou sur une zone perméable à la lumière de la surface d'appui. Par « lumière », on entend une lumière ayant au moins une longueur d'onde appartenant au spectre du visible. Les moyens de détection de lumière peuvent comprendre plusieurs capteurs de lumière répartis angulairement autour de l'orifice de distribution pour évaluer l'intensité de la lumière à plusieurs endroits de l'orifice de distribution, par exemple des paires de capteurs de lumière diamétralement opposés par rapport à l'orifice de distribution.

- Les moyens de détection d'un contact comprennent au moins une électrode reliée à un circuit électrique, l'électrode étant disposée sur la surface d'appui, le circuit électrique étant apte à détecter une modification d'au moins une valeur choisie parmi la tension, l'intensité, la résistance électrique, lorsque la peau d'un sujet est en contact avec l'électrode. La peau étant un conducteur électrique ayant une résistance électrique déterminée, lorsqu'elle se met en contact avec la surface d'appui, le circuit électrique détecte une modification caractéristique de la résistance électrique de la peau justifiant son contact contre la surface d'appui. Avantageusement, les électrodes sont des capteurs capacitifs. De préférence, on dispose deux paires d'électrodes ou plus pour avoir plus de précisions.

- Les moyens de détection d'un contact comprennent des moyens de détection d'une pression minimale exercée sur la surface d'appui. Lorsqu'un sujet place l'organe de réception à disposition de l'orifice de distribution, il appuie la peau contre la surface d'appui et exerce une pression *(i.e.* force) sur cette dernière. La détection de cette pression donne une information non seulement sur la présence du contact mais en outre sur un appui exercé. Or le fait d'appuyer permet de distribuer la goutte de façon plus stable ou plus assurée. Les moyens de détection d'une pression minimale comprennent par exemple un capteur de pression configuré pour donner une information, de préférence quantitative, sur la pression exercée sur la surface d'appui.

― Les moyens de détection d'une pression minimale comprennent une première chambre d'air déformable lorsque la surface d'appui est en appui contre la peau du sujet, et au moins un capteur de pression apte à être activé lorsque la pression de la première chambre d'air atteint un seuil prédéterminé. La pression détectée peut être une pression d'air dans la première chambre d'air ou une pression de contact *(i.e.* force) subie par la première chambre d'air. Dans le premier cas, cela peut être l'appui de la peau contre la surface d'appui qui entraine une variation de la pression d'air dans la première chambre d'air jusqu'à atteindre le seuil prédéterminé. Dans le second cas, cela peut être l'appui de la peau contre la surface d'appui qui entraine un contact entre la surface d'appui et les moyens de détection d'une pression minimale par déformation de la première chambre d'air jusqu'à ce que la pression ou la force atteigne le seuil prédéterminé. La première chambre d'air peut comprendre un appendice permettant de faciliter le contact entre la surface d'appui et les moyens de détection d'une pression minimale. On comprend que la chambre d'air est étanche à l'air et que l'enveloppe de la chambre d'air est avantageusement déformable élastiquement.

― les moyens de détection d'une pression minimale comprennent une seconde chambre d'air de contenance inférieure à celle de la première chambre d'air, la seconde chambre d'air étant disposée entre la première chambre d'air et le capteur de pression pour concentrer les efforts sur le capteur de pression. La seconde chambre d'air permet de répartir de manière plus homogène un appui local de la peau contre la surface d'appui. Cela permet de rendre plus sensibles les moyens de détection d'une pression minimale et moins dépendants du lieu d'exercice de l'appui. La mesure de la pression de la première chambre d'air se fait alors par l'intermédiaire de la seconde chambre d'air.

― Les moyens de détection de pression minimale comprennent deux électrodes reliées à une source d'électricité et une partie électriquement conductrice montée mobile entre une position de circuit ouvert, dans laquelle un circuit électrique formé par la source d'électricité et les électrodes est ouvert et une position de circuit fermé dans laquelle la partie électriquement conductrice relie électriquement les deux électrodes, la partie électriquement conductrice se déplaçant entre les deux positions lorsqu'une pression minimale est exercée sur la surface d'appui. Ainsi, lorsque la surface d'appui s'appuie contre la peau, la pression appliquée déplace la partie électriquement conductrice vers la position de circuit fermé. Le contact entre la surface d'appui et la peau est dans ce cas détecté par la circulation d'un courant électrique. La partie électriquement conductrice est mobile entre les deux positions par exemple par déformation élastique ou par translation.

― Les moyens de détection de pression minimale comprennent un élément de rappel disposé entre la partie électriquement conductrice et les électrodes et tendant à rappeler la partie électriquement conductrice en position de circuit ouvert, l'élément de rappel étant par exemple un ressort ou une chambre d'air disposée entre la partie électriquement conductrice et les électrodes. L'appui de la peau contre la surface d'appui peut déformer l'élément de rappel, faisant ainsi passer la partie électriquement conductrice vers sa position de circuit fermé, indiquant la détection du contact. Lorsque la peau quitte la surface d'appui, l'élément de rappel reprend sa position de repos, ramenant la partie électriquement conductrice vers sa position de circuit ouvert.

L'invention concerne en outre un kit de distribution d'un produit liquide dans un organe de réception d'un sujet, comprenant un dispositif de distribution du produit liquide et un dispositif d'assistance décrit ci-dessus. Le kit de distribution permet de distribuer une goutte de liquide dans l'organe de réception de manière simple et précise. De préférence le dispositif d'assistance et le dispositif de distribution sont des dispositifs distincts, rapportés l'un sur l'autre et amovibles, mais on peut envisager qu'ils soient d'un seul bloc, venus de matière.

Nous allons maintenant présenter des modes de réalisation particuliers de l'invention donnés à titre d'exemples non limitatifs et à l'appui des figures annexées sur lesquelles :
- la figure 1 est un ensemble de deux vues, respectivement en perspective éclatée à gauche et en perspective assemblée à droite, d'un dispositif de distribution solidarisé à un dispositif d'assistance selon un premier mode de réalisation,
- la figure 2 est un ensemble similaire à celui de la figure 1 d'un dispositif de distribution solidarisé à un dispositif d'assistance, selon un deuxième mode de réalisation,
- la figure 3A est un ensemble similaire à celui de la figure 1 d'un dispositif de distribution solidarisé à un dispositif d'assistance, selon un troisième mode de réalisation,
- la figure 3B est une vue en coupe longitudinale (la direction longitudinale correspondant à celle de l'axe de distribution des gouttes) schématique d'une partie supérieure du dispositif d'assistance selon une variante du troisième mode de réalisation,
- la figure 3C est une vue en coupe longitudinale schématique d'une partie supérieure d'un dispositif d'assistance selon une autre variante du troisième mode de réalisation,
- la figure 4A est un ensemble similaire à celui de la figure 1 d'un dispositif de distribution solidarisé à un dispositif d'assistance selon un quatrième mode de réalisation,
- la figure 4B est une vue en coupe longitudinale schématique d'une partie supérieure du dispositif d'assistance selon le quatrième mode de réalisation,
- la figure 5A est un ensemble similaire à celui de la figure 1 d'un dispositif de distribution solidarisé à un dispositif d'assistance selon un cinquième mode de réalisation,
- la figure 5B est une vue en coupe longitudinale schématique d'une partie supérieure du dispositif d'assistance selon le cinquième mode de réalisation.

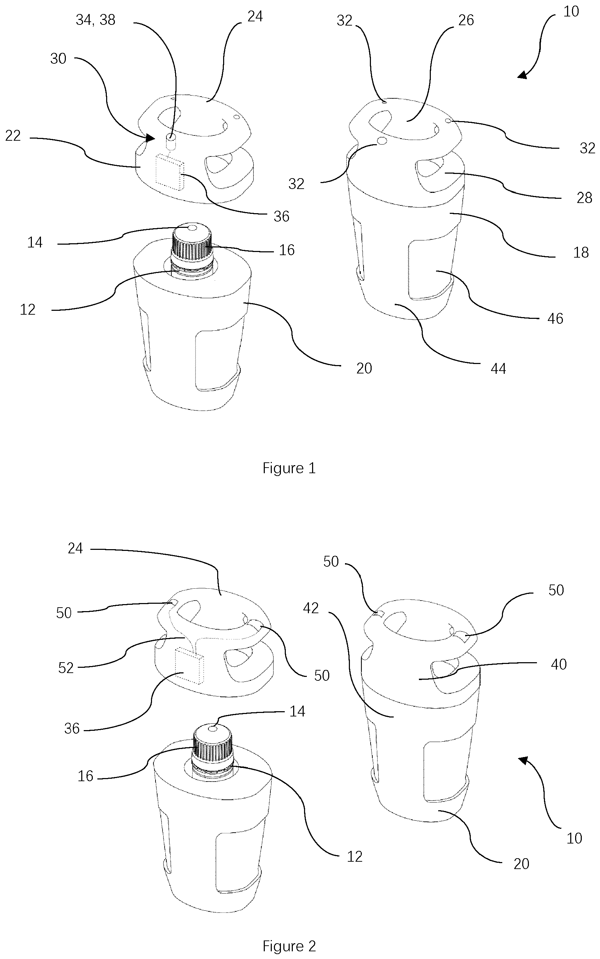

La figure 1 illustre un dispositif d'assistance 10 à l'utilisation d'un dispositif de distribution 12 d'un produit liquide sous forme de gouttes dans un organe de réception d'un sujet selon un premier mode de réalisation.

Le dispositif de distribution 12 comprend ici un réservoir et un embout de distribution pourvu d'un orifice de distribution de gouttes 14 protégé par un capuchon 16, par exemple vissé sur l'embout de distribution. Sur les figures, la référence 14 indique l'emplacement orifice de l'orifice distribution 14 recouvert par le capuchon 16. Le dispositif d'assistance 10 comprend des moyens de solidarisation 18 au dispositif de distribution 12 disposé à l'intérieur de celui-ci, de sorte que les deux dispositifs d'assistance 10, et de distribution 12 forment un ensemble solidaire. Les moyens de solidarisation 18 peuvent comprendre par exemple un encliquetage du réservoir dans le dispositif d'assistance 10. Dans une variante parmi d'autres, l'embout de distribution pourvu de l'orifice de distribution 14 pourrait faire partie du dispositif d'assistance 10, rapporté sur le réservoir du dispositif de distribution 12 au moment de la solidarisation des deux dispositifs d'assistance 10, et de distribution 12.

Le dispositif d'assistance 10 comprend un corps principal 20 dans lequel on place le dispositif de distribution 12, et une structure d'appui 22 comportant une surface d'appui 24 contre la peau de l'utilisateur lors de la distribution de gouttes dans un organe de réception, par exemple un oeil. La structure d'appui 22 est montée amovible sur le corps principal 20 pour permettre de positionner et solidariser le dispositif de distribution 12 à l'intérieur du dispositif d'assistance 10, éventuellement au moyen d'une charnière, ou encore en étant intégralement détachable. La structure d'appui 22 peut être conçue de manière assez souple, en particulier au niveau de la surface d'appui 24 pour assurer le confort de l'appui contre la peau de l'utilisateur et s'adapter aux reliefs au voisinage de l'organe cible, et/ou assez rigide pour assurer la fonction de support à l'appui et imposer une distance prédéterminée entre l'orifice de distribution 14 et l'organe cible. La surface d'appui 24 comprend un orifice axial 26 destiné à autoriser le passage de gouttes de produit liquide de l'orifice de distribution 14 vers l'organe de réception. La structure d'appui 22 comprend, de manière facultative, des ouvertures traversantes 28 sur deux côtés opposés et à son extrémité, notamment pour éviter que l'œil de l'utilisateur soit dans le noir lorsque le dispositif d'assistance 10 est appliqué contre la peau de l'utilisateur autour de son œil, ou encore pour rendre certaines parties de la surface d'appui 24 plus souples pour améliorer le confort du sujet.

Le dispositif d'assistance 10 comprend des moyens d'écartement configurés pour préserver une distance prédéterminée entre la surface d'appui 24 et l'orifice de distribution 14. Les moyens d'écartement peuvent être une partie rigide de la structure d'appui 22 qui maintient la surface d'appui 24 à distance de l'orifice de distribution 14. La structure d'appui 22 étant montée amovible sur le corps principal 20, elle peut servir de moyens de réglage de la surface d'appui 24, de façon à pouvoir faire varier la distance maintenue entre l'orifice de distribution 14 et au moins une partie de la surface d'appui 24 en changeant au moins une partie de la structure d'appui 22. Dans une variante, les moyens de réglage peuvent comprendre une vis sans fin ou un élément flexible qui peut se verrouiller à des distances prédéterminées.

Le dispositif d'assistance 10 comprend des moyens de détection d'un contact entre la surface d'appui et la peau du sujet. Les moyens de détection d'un contact comprennent ici des moyens de détection de lumière 30 configurés pour détecter une variation de l'intensité de la lumière sur une partie de la surface d'appui 24. Les moyens de détection de lumière 30 comprennent dans cet exemple trois tunnels 32 débouchant sur la surface d'appui 24, répartis angulairement sur la surface d'appui 24. Dans chaque tunnel 32 est disposé un capteur de lumière 34 relié de préférence à un système de traitement 36 de l'information fournie par les capteurs de lumière 34. Ainsi, lorsqu'un sujet place sa peau contre la surface d'appui 24, la lumière ne pénètre plus dans les tunnels 32. Les capteurs de lumière 34 détectent donc une variation de l'intensité de la lumière qui indique que le liquide est prêt à être distribué. On notera que le nombre de tunnels 32 n'est pas limité à trois. Ils peuvent être configurés et positionnés en fonction du besoin.

Dans une variante, les moyens de détection de lumière 30 comprennent des capteurs de lumière 34 disposés directement sur la surface d'appui 24 ou sous une partie perméable à la lumière de celle-ci. En effet, au moins une partie de la surface d'appui 24 peut être fabriquée en un matériau perméable à la lumière qui est détectable par les capteurs de lumière 34.

Le dispositif d'assistance 10 comprend en outre des moyens de détection d'une couleur 38 (visibles sur la figure 1) d'une zone disposée face à la surface d'appui et/ou l'orifice de distribution des gouttes 14. Comme les moyens de détection de lumière 30, Les moyens de détection d'une couleur 38 comprennent un ou plusieurs capteurs de couleurs disposés sur ou au voisinage de la surface d'appui 24 et configurés pour être orientés vers l'organe de réception lorsque le dispositif d'assistance 10 est en appui contre la peau du sujet, aptes à capter une ou plusieurs couleurs déterminées caractérisantes de l'organe de réception et à fournir un signal positif ou négatif en fonction de la détection. Dans une variante, les capteurs de couleurs détectent une ou plusieurs couleurs de l'objet placé en face de ceux-ci et envoient l'information au système de traitement 36 qui les compare à au moins une couleur ou une gamme de couleurs prédéterminées caractérisantes de l'organe de réception.

Le dispositif d'assistance 10 comporte des moyens de verrouillage de la distribution de produit aptes à n'autoriser l'activation du dispositif de distribution 12 qu'en fonction du contact détecté et/ou d'une couleur détectée. Les moyens de verrouillage comprennent par exemple une soupape disposée dans l'embout de distribution, apte à bloquer la distribution de liquide si un contact entre la peau et la surface d'appui 24 n'est pas détecté par les moyens de détection de lumière 30 et/ou si une couleur prédéterminée n'est pas détectée par les moyens de détection d'une couleur 38.

Le dispositif d'assistance 10 comporte des moyens optiques 40 destinés à être disposés au voisinage de l'orifice de distribution 14 et configurés pour fournir une information sur la distribution d'une goutte du produit liquide par le dispositif de distribution 12. Les moyens optiques 40 comprennent un émetteur et un récepteur d'un signal optique, configurés pour détecter la présence d'une goutte perturbant le signal optique et pour mesurer la durée de cette présence. L'émetteur comprend par exemple des diodes émettrices de rayons infrarouges et le récepteur comprend par exemple des photo-transistors aptes à détecter des rayons infrarouges. L'émetteur et le récepteur détectent la présence d'une goutte traversant le signal optique lorsque les rayons optiques sont perturbés, par exemple par une variation de l'intensité des rayons. L'émetteur et le récepteur sont de préférence situés à une distance comprise entre 1 et 3 mm, préférentiellement 2 mm, de l'orifice de distribution 14.

Le dispositif d'assistance 10 comprend en outre des moyens de mesure de l'inclinaison 42 configurés pour fournir une information sur l'inclinaison du dispositif de distribution 12 solidarisé au dispositif d'assistance 10. Les moyens de mesure de l'inclinaison 42 comprennent par exemple un inclinomètre tel qu'un gyroscope électronique ou un accéléromètre. L'inclinomètre est de préférence placé dans le corps principal 20, par exemple dans une zone destinée à être placée au voisinage de l'embout de distribution.

Le dispositif d'assistance 10 comprend des moyens de mesure du poids 44 du dispositif de distribution 12 solidarisé au dispositif d'assistance 10, configurés pour fournir une information sur la quantité de produit liquide restant dans le dispositif de distribution 12. Les moyens pour mesurer le poids 44 comprennent un capteur de poids, par exemple de type capteur de pression (« Force Sensing Resistor » ou FSR en anglais), disposé en dessous du réservoir pour peser le dispositif de distribution 14 et en déduire le poids, et donc le volume, de la quantité de liquide restant dans le réservoir. Le capteur de poids 44 peut être situé en-dessous ou au-dessus du réservoir. Dans une variante, le dispositif d'assistance 10 présente plusieurs capteurs de poids autour du réservoir afin de pouvoir mesurer le poids du dispositif de distribution 12 solidarisé au dispositif d'assistance 10 quelle que soit son inclinaison.

Les moyens optiques 40, les moyens de mesure de l'inclinaison 42 et les moyens de mesure du poids 44 transmettent les informations au système de traitement 36 qui les utilise, et/ou les combine avec d'autres informations, pour estimer la quantité de produit liquide distribuée, plus particulièrement le volume de la goutte distribuée. Pour cela, le système de traitement 36 calcule d'abord un volume théorique de goutte à partir d'informations sur la viscosité du produit liquide et/ou les caractéristiques géométriques de l'orifice de distribution 14, voire sur d'autres caractéristiques de l'embout de distribution et/ou du dispositif de distribution 12. Puis, il pondère ce volume théorique en prenant en compte l'information fournie par les moyens ci-dessus, par exemple en calculant un coefficient lié à un ou plusieurs paramètres tels que l'intensité de la pression d'activation appliquée par l'utilisateur sur le réservoir pour provoquer la formation de la goutte, le profil de variation de cette pression d'activation dans le temps, la durée de perturbation du signal optique fournie par les moyens optiques 40, l'inclinaison du dispositif de distribution 12 solidarisé au dispositif d'assistance 10 par rapport à un axe horizontal, la mesure du poids du dispositif de distribution 12.

Le dispositif d'assistance 10 comporte également une zone de préhension 46 destinée ici à la fois à la préhension et à permettre l'application d'une pression par l'utilisateur pour distribuer le produit liquide. La zone de préhension 46 est disposée sur deux côtés opposés du corps principal 20. Une pression d'activation exercée sur la zone de préhension 46 est transmise au réservoir du dispositif de distribution 12 pour distribuer le produit liquide. La zone de préhension 46 peut être réalisée en un matériau différent, en particulier plus souple, que celui du reste du corps principal 20. Elle peut également comprendre des reliefs qui facilitent la préhension par l'utilisateur. Par ailleurs, grâce à la présence de la zone de préhension 46, le dispositif d'assistance 10 augmente la surface de préhension de l'utilisateur et de pression d'activation sur le réservoir par rapport à celle du dispositif de distribution 12 seul, ce qui est particulièrement avantageux pour des utilisateurs atteints de maladies neuromusculaires.

La figure 2 illustre un dispositif d'assistance 10 à l'utilisation d'un dispositif de distribution 12 d'un produit liquide sous forme de gouttes dans un organe de réception d'un sujet selon un deuxième mode de réalisation. Le dispositif d'assistance 10 de la figure 2 diffère de celui de la figure 1 principalement par les moyens de détection d'un contact. Le reste de la structure est sensiblement similaire et n'est pas décrit. Les mêmes références numériques sont utilisées dans la suite de la description pour désigner les éléments en commun.

Dans ce deuxième mode de réalisation, les moyens de détection d'un contact comprennent deux électrodes 50 reliée à un circuit électrique 52. Les électrodes 50 sont disposées de manière diamétralement opposée sur la surface d'appui 24. Elles peuvent être placées sous une couche couverture électriquement conductrice pour améliorer l'aspect esthétique. Les électrodes 50 sont avantageusement des capteurs capacitifs configurés pour générer un courant électrique dans le circuit 52 lorsque un objet ayant des propriétés électriques, en particulier une résistance électrique, similaires à celles de la peau.

Ainsi, le circuit électrique 52 est apte à détecter une modification d'au moins une valeur choisie parmi la tension, l'intensité, la résistance électrique, lorsque la peau d'un sujet est en contact avec les électrodes 50. Pour cela, le circuit électrique 52 comprend des fils conducteurs, une source d'électricité, et d'autres composants électroniques tels qu'un voltmètre ou un ampèremètreLe circuit électrique 52 est relié au système de traitement 36 qui reçoit l'information sur le contact entre la peau et la surface d'appui 24 pour déclencher ou non la distribution de liquide.

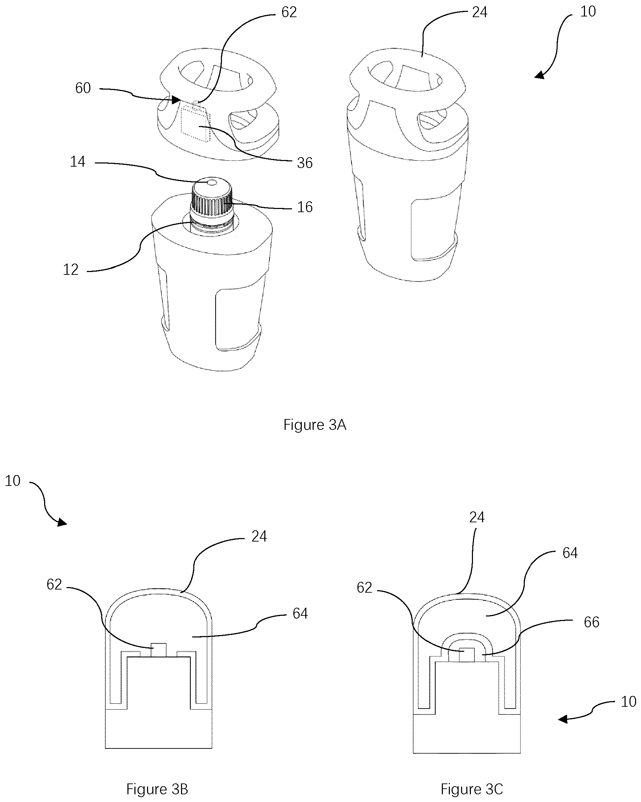

Les figures 3A, 3B et 3C illustrent un dispositif d'assistance 10 à l'utilisation d'un dispositif de distribution 12 d'un produit liquide sous forme de gouttes dans un organe de réception d'un sujet selon respectivement un troisième mode de réalisation et deux variantes de ce troisième mode de réalisation. Le dispositif d'assistance 10 des figures 3A, 3B et 3C diffère de celui de la figure 1 principalement par les moyens de détection d'un contact.

Les moyens de détection d'un contact de la figure 3A comprennent des moyens de détection d'une pression minimale 60 exercée sur la surface d'appui 24. Les moyens de détection d'une pression minimale 60 comprennent ici un capteur de pression 62 ayant une branche s'étendant jusqu'à la surface d'appui 24 ou au contact d'une couche de matériau déformable de la surface d'appui 24. Lorsqu'un sujet appuie sa peau contre la surface d'appui 24, il exerce une pression (i.e. force), supérieure à la pression minimale prédéfinie, sur cette dernière. La détection de cette pression donne une information sur la présence du contact entre la peau et la surface d'appui 24.

Les moyens de détection d'un contact de la figure 3B comprennent en plus une première chambre d'air déformable 64 entre la surface d'appui 24 et le capteur de pression 62. La première chambre d'air 64 comprend une enveloppe élastique étanche remplie d'air ou d'un autre type de fluide. L'enveloppe peut supporter la surface d'appui 24 sur une face extérieure. La première chambre d'air 64 se déforme lorsque la peau du sujet est en appui contre la surface d'appui 24. Lorsque la pression d'air de la première chambre d'air 64 dépasse un seuil prédéterminé, le capteur de pression 62 est activé et fournit une information sur le bon positionnement de l'organe de réception par rapport à l'orifice de distribution 14. Dans ce cas, le capteur de pression 62 est configuré pour détecter la pression d'air. Alternativement, le capteur de pression 62 est activé lorsque la surface d'appui 24, sous la pression d'appui de la peau, se déplace vers le capteur de pression 62 par déformation de la première chambre d'air 64 jusqu'à exercer une pression supérieure au seuil prédéterminé. Dans ce cas, le capteur de pression 62 est configuré pour détecter une pression (i.e. force) de contact. Le contact entre la surface d'appui 24 et le capteur de pression 62 peut être aidé par un appendice disposé dans la première chambre d'air 64.

Les moyens de détection d'un contact de la figure 3C comprennent une seconde chambre d'air 66 de contenance inférieure à celle de la première chambre d'air 64. La seconde chambre d'air 66 est disposée entre la première chambre d'air 64 et le capteur de pression 62 pour concentrer les efforts sur ce dernier. Lorsque le sujet positionne l'organe de réception face à l'orifice de distribution 14, sa peau s'appuie contre la surface d'appui 24, déformant d'abord la première chambre d'air 64 puis la deuxième chambre d'air 66 pour exercer une pression sur le capteur de pression 62 directement par contact ou en comprimant l'air de la deuxième chambre d'air 66 dans laquelle est située le capteur de pression 62. Le dispositif d'assistance est apte à traiter l'information lorsque la pression dépasse le seuil prédéterminé, par exemple pour autoriser la distribution de produit liquide.

Dans les trois figures, les moyens de détection d'une pression minimale 60 sont reliés au système de traitement 36 pour lui fournir une information sur le bon positionnement de l'organe de réception face à l'orifice de distribution 14 et/ou une information quantitative sur la pression exercée sur la surface d'appui 24.

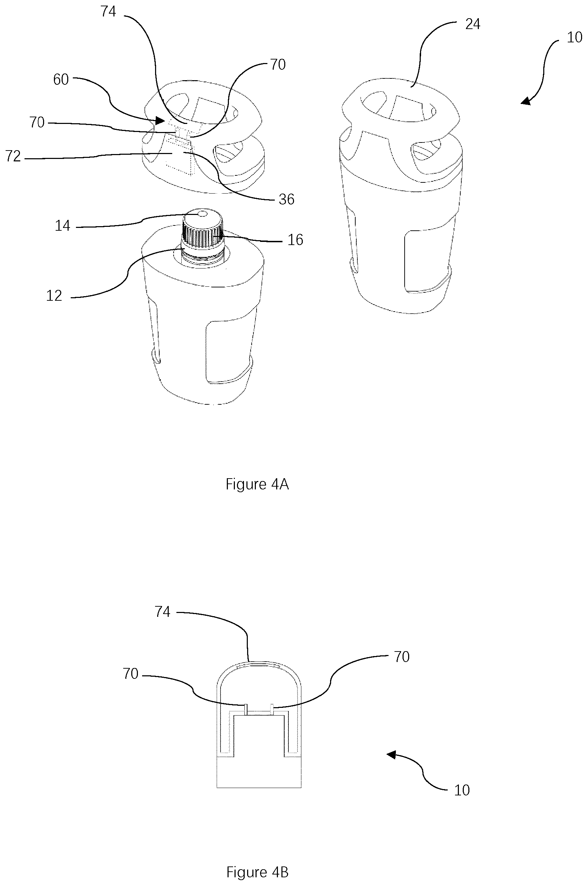

Les figures 4A et 4B illustrent un dispositif d'assistance 10 à l'utilisation d'un dispositif de distribution 12 d'un produit liquide sous forme de gouttes dans un organe de réception d'un sujet selon un quatrième mode de réalisation. Le dispositif d'assistance 10 des figures 4A et 4B diffère de celui de la figure 1 principalement par les moyens de détection d'un contact.

Les moyens de détection de pression minimale 60 comprennent deux électrodes 70 reliées à une source d'électricité 72 et une partie électriquement conductrice 74. Les électrodes 70 sont placées dans la structure d'appui 22, à distance de la surface d'appui 24. La partie électriquement conductrice 74, par exemple une plaque métallique, est solidaire à au moins une partie de la surface d'appui 24. La partie électriquement conductrice 74 est montée mobile entre une position de circuit ouvert, dans laquelle un circuit électrique formé par la source d'électricité et les électrodes est ouvert et une position de circuit fermé dans laquelle la partie électriquement conductrice relie électriquement les deux électrodes 70. Lorsque le sujet place l'organe de réception en face de l'orifice de distribution 14, il appuie sa peau contre la surface d'appui 24 qui, sous la pression d'appui et étant flexible, se déforme et déplace la partie électriquement conductrice 74, qui lui est solidaire, de la position de circuit ouvert à la position de circuit fermé. Lorsque le circuit est fermé, un courant y circule et la détection de ce courant indique le contact entre le bon positionnement de l'organe de réception qui est prêt pour recevoir la distribution de liquide.

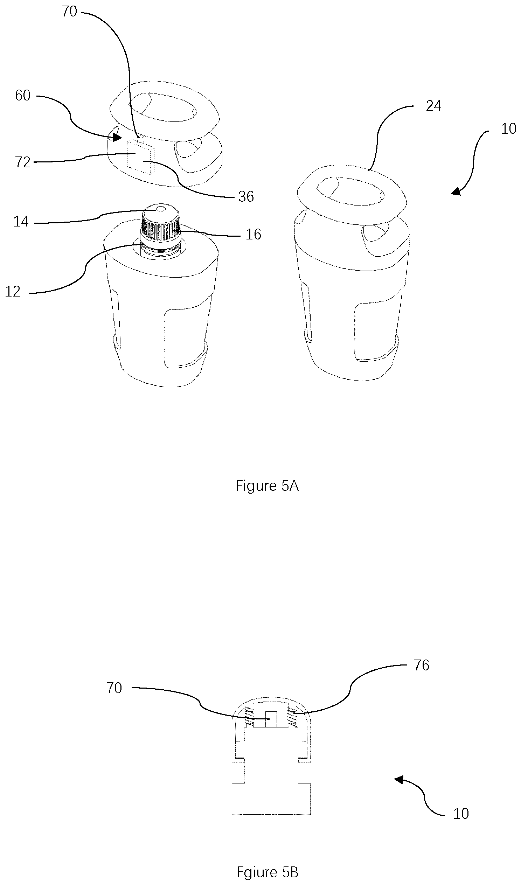

Les figures 5A et 5B illustrent un dispositif d'assistance 10 à l'utilisation d'un dispositif de distribution 12 d'un produit liquide sous forme de gouttes dans un organe de réception d'un sujet selon un cinquième mode de réalisation. Le dispositif d'assistance 10 des figures 5A et 5B est une variante de celui des figures 4A et 4B.

Les moyens de détection de pression minimale 60 comprennent un élément de rappel 76, par exemple un ressort, entre chacune des électrodes 70 et la partie électriquement conductrice 74. L'élément de rappel 76 se déforme, par exemple en se comprimant, lorsque la partie électriquement conductrice 74 se rapproche des électrodes 70 sous la pression de la peau qui s'appuie sur la surface d'appui 24. Une fois que la peau se détache de la surface d'appui 24, la disparition de la pression libère l'élément de rappel 76 qui reprend sa position non déformée, entrainant avec lui la partie électriquement conductrice 74.

L'invention n'est pas limitée aux modes de réalisation présentés et d'autres modes de réalisation apparaîtront clairement à l'homme du métier.

## Revendications

1. Dispositif d'assistance (10) à l'utilisation d'un dispositif de distribution (12) d'un produit liquide sous forme de gouttes dans un organe de réception d'un sujet, le dispositif de distribution (12) comprenant un réservoir, le dispositif d'assistance (10) comprenant :
- des moyens de solidarisation (18) au dispositif de distribution,
- une surface d'appui (24) contre la peau du sujet, disposée au voisinage d'un orifice de distribution (14) du produit liquide,
**caractérisé en ce que** le dispositif d'assistance comprend
- des moyens de détection d'un contact entre la surface d'appui (24) et la peau du sujet.

2. Dispositif d'assistance (10) selon la revendication précédente, comprenant des moyens d'écartement configurés pour préserver une distance prédéterminée entre la surface d'appui (24) et l'orifice de distribution des gouttes (14).

3. Dispositif d'assistance (10) selon l'une quelconque des revendications précédentes, comprenant en outre des moyens de détection d'une couleur (38) d'une zone disposée face à la surface d'appui (24) et/ou l'orifice de distribution des gouttes (14), et éventuellement un système de traitement des informations (36) pour comparer la couleur détectée à au moins une couleur prédéterminée.

4. Dispositif d'assistance (10) selon l'une quelconque des revendications précédentes, comprenant en outre des moyens de verrouillage de la distribution de produit aptes à n'autoriser l'activation du dispositif de distribution (12) qu'en fonction du contact détecté et/ou d'une couleur détectée.

5. Dispositif d'assistance (10) selon l'une quelconque des revendications précédentes, comprenant en outre des moyens optiques (40) destinés à être disposés au voisinage de l'orifice de distribution des gouttes (14) et configurés pour fournir une information sur la distribution d'une goutte du produit liquide par le dispositif de distribution (12).

6. Dispositif d'assistance (10) selon la revendication précédente, comprenant en outre des moyens de mesure de l'inclinaison (42) configurés pour fournir une information sur l'inclinaison du dispositif de distribution (12) solidarisé au dispositif d'assistance (10), et un système de traitement des informations (36) sur la distribution d'une goutte et sur l'inclinaison du dispositif de distribution (12) solidarisé au dispositif d'assistance (10) pour fournir une information sur la quantité de produit liquide distribuée.

7. Dispositif d'assistance (10) selon l'une quelconques des revendications précédentes, comprenant des moyens de mesure du poids (44) du dispositif de distribution (12) solidarisé au dispositif d'assistance (10), configurés pour fournir une information sur la quantité de produit liquide restant dans le dispositif de distribution (12).

8. Dispositif d'assistance (10) selon l'une quelconque des revendications précédentes, comprenant des moyens de réglage de la surface d'appui (24), de façon à pouvoir faire varier la distance maintenue entre l'orifice de distribution (14) et au moins une partie de la surface d'appui (24).

9. Dispositif d'assistance (10) selon l'une quelconque des revendications précédentes, dans lequel les moyens de détection d'un contact comprennent des moyens de détection de lumière (30) configurés pour détecter une variation de l'intensité de la lumière sur une partie de la surface d'appui (24).

10. Dispositif d'assistance (10) selon l'une quelconque des revendications précédentes, dans lequel les moyens de détection d'un contact comprennent au moins une électrode (50, 70) reliée à un circuit électrique (52), l'électrode (50, 70) étant disposée sur la surface d'appui (24), le circuit électrique (52) étant apte à détecter une modification d'au moins une valeur choisie parmi la tension, l'intensité, la résistance électrique, lorsque la peau d'un sujet est en contact avec l'électrode (50, 70).

11. Dispositif d'assistance (10) selon l'une quelconque des revendications précédentes, dans lequel les moyens de détection d'un contact comprennent des moyens de détection d'une pression minimale (60) exercée sur la surface d'appui (24).

12. Dispositif d'assistance (10) selon la revendication précédente, dans lequel les moyens de détection d'une pression minimale (60) comprennent une première chambre d'air déformable (64) lorsque la surface d'appui (24) est en appui contre la peau du sujet, et au moins un capteur de pression (62) apte à être activé lorsque la pression de la première chambre d'air (64) atteint un seuil prédéterminé.

13. Dispositif d'assistance (10) selon la revendication précédente, dans lequel les moyens de détection d'une pression minimale (60) comprennent une seconde chambre d'air (66) de contenance inférieure à celle de la première chambre d'air (64), la seconde chambre d'air (66) étant disposée entre la première chambre d'air (64) et le capteur de pression (62) pour concentrer les efforts sur le capteur de pression (62).

14. Dispositif d'assistance (10) selon l'une quelconque des revendications 11 à 13, dans lequel les moyens de détection de pression minimale (60) comprennent deux électrodes (70) reliées à une source d'électricité (72) et une partie électriquement conductrice (74) montée mobile entre une position de circuit ouvert, dans laquelle un circuit électrique formé par la source d'électricité et les électrodes (70) est ouvert et une position de circuit fermé dans laquelle la partie électriquement conductrice (74) relie électriquement les deux électrodes (70), la partie électriquement conductrice (74) se déplaçant entre les deux positions lorsqu'une pression minimale est exercée sur la surface d'appui (24).

15. Dispositif d'assistance (10) selon la revendication précédente, dans lequel les moyens de détection de pression minimale (60) comprennent un élément de rappel (76) disposé entre la partie électriquement conductrice (74) et les électrodes (70) et tendant à rappeler la partie électriquement conductrice (74) en position de circuit ouvert, l'élément de rappel (76) étant par exemple un ressort ou une chambre d'air disposée entre la partie électriquement conductrice (74) et les électrodes (70).

16. Kit de distribution d'un produit liquide dans un organe de réception d'un sujet, comprenant un dispositif de distribution du produit liquide (12) et un dispositif d'assistance (10) selon l'une quelconque des revendications précédentes.

## Patentansprüche

1. Hilfsvorrichtung (10) zur Verwendung einer Vorrichtung (12) zur Abgabe eines flüssigen Produkts in Form von Tropfen in ein aufnehmendes Organ eines Patienten, wobei die Abgabevorrichtung (12) ein Reservoir aufweist, wobei die Hilfsvorrichtung (10) aufweist:
- Mittel (18) zur Befestigung an der Abgabevorrichtung,
- eine Fläche (24) zur Anlage an die Haut des Patienten, die in der Nähe einer Abgabeöffnung (14) für das flüssige Produkt angeordnet ist,
**dadurch gekennzeichnet, dass** die Hilfsvorrichtung Mittel zur Erfassung des Kontakts zwischen der Anlagefläche (24) und der Haut der Person aufweist.

2. Hilfsvorrichtung (10) nach dem vorhergehenden Anspruch, die Abstandsmittel aufweist, die so konfiguriert sind, dass sie einen vorbestimmten Abstand zwischen der Anlagefläche (24) und der Tropfenabgabeöffnung (14) aufrechterhalten.

3. Hilfsvorrichtung (10) nach einem der vorhergehenden Ansprüche, die ferner Mittel zum Erfassen einer Farbe (38) eines Bereichs, der gegenüber der Anlagefläche (24) und/oder der Tropfenabgabeöffnung (14) angeordnet ist, und optional ein System (36) zur Verarbeitung von Informationen (36) zum Vergleichen der erfassten Farbe mit mindestens einer vorbestimmten Farbe aufweist.

4. Hilfsvorrichtung (10) nach einem der vorhergehenden Ansprüche, die ferner eine Produktabgabe-Sperreinrichtung aufweist, die so beschaffen ist, dass sie die Aktivierung der Abgabevorrichtung (12) nur in Abhängigkeit von der erfassten Berührung und/oder einer erfassten Farbe ermöglicht.

5. Hilfsvorrichtung (10) nach einem der vorhergehenden Ansprüche, die ferner optische Mittel (40) aufweist, die dazu bestimmt sind, in der Nähe der Tropfenabgabeöffnung (14) angeordnet zu werden, und so konfiguriert sind, dass sie eine Information über die Abgabe eines Tropfens des flüssigen Produkts durch die Abgabevorrichtung (12) liefern.

6. Hilfsvorrichtung (10) nach dem vorhergehenden Anspruch, die ferner eine Neigungsmesseinrichtung (42), die so konfiguriert ist, dass sie eine Information über die Neigung der an der Hilfsvorrichtung (10) angebrachten Abgabevorrichtung (12) liefert, und ein System zur Verarbeitung von Informationen (36) über die Abgabe eines Tropfens und über die Neigung der an der Hilfsvorrichtung (10) angebrachten Abgabevorrichtung (12) aufweist, um eine Information über die Menge des abgegebenen flüssigen Produkts zu liefern.

7. Hilfsvorrichtung (10) nach einem der vorhergehenden Ansprüche, die eine an der Hilfsvorrichtung (10) angebrachte Gewichtsmesseinrichtung (44) der Abgabevorrichtung (12) aufweist, die so konfiguriert ist, dass sie eine Information über die in der Abgabevorrichtung (12) verbleibende Menge an flüssigem Produkt liefert.

8. Hilfsvorrichtung (10) nach einem der vorhergehenden Ansprüche, die Mittel zum Einstellen der Anlagefläche (24) aufweist, um den zwischen der Abgabeöffnung (14) und mindestens einem Teil der Anlagefläche (24) eingehaltenen Abstand verändern zu können.

9. Hilfsvorrichtung (10) nach einem der vorhergehenden Ansprüche, wobei das Mittel zum Erkennen eines Kontakts ein Lichterkennungsmittel (30) aufweist, das so konfiguriert ist, dass es eine Veränderung der Lichtintensität auf einem Teil der Anlagefläche (24) erkennt.

10. Hilfsvorrichtung (10) nach einem der vorhergehenden Ansprüche, wobei die Mittel zum Erfassen eines Kontakts mindestens eine Elektrode (50, 70) aufweisen, die mit einer elektrischen Schaltung (52) verbunden ist, wobei die Elektrode (50, 70) auf der Anlagefläche (24) angeordnet ist, wobei die elektrische Schaltung (52) eingerichtet ist, eine Änderung mindestens eines Werts zu erfassen, der aus Spannung, Strom und elektrischem Widerstand ausgewählt ist, wenn die Haut eines Patienten in Kontakt mit der Elektrode (50, 70) ist.

11. Hilfsvorrichtung (10) nach einem der vorhergehenden Ansprüche, wobei die Mittel zum Erfassen eines Kontakts Mittel zum Erfassen eines auf die Anlagefläche (24) ausgeübten Mindestdrucks (60) aufweisen.

12. Hilfsvorrichtung (10) nach dem vorhergehenden Anspruch, wobei die Mittel zum Erfassen eines Mindestdrucks (60) eine erste verformbare Luftkammer (64), wenn die Anlagefläche (24) an der Haut des Patienten anliegt, und mindestens einen Drucksensor (62) aufweisen, der eingerichtet ist, aktiviert zu werden, wenn der Druck der ersten Luftkammer (64) einen vorbestimmten Schwellenwert erreicht.

13. Hilfsvorrichtung (10) nach dem vorhergehenden Anspruch, wobei die Mittel zum Erfassen eines Mindestdrucks (60) eine zweite Luftkammer (66) mit kleinerem Fassungsvermögen als die erste Luftkammer (64) aufweist, wobei die zweite Luftkammer (66) zwischen der ersten Luftkammer (64) und dem Drucksensor (62) angeordnet ist, um die Kräfte auf den Drucksensor (62) zu konzentrieren.

14. Hilfsvorrichtung (10) nach einem der Ansprüche 11 bis 13, wobei die Mittel zum Erfassen eines Mindestdrucks (60) zwei Elektroden (70), die mit einer Stromquelle (72) verbunden sind, und einen elektrisch leitenden Abschnitt (74) aufweist, der beweglich zwischen einer Position mit offenem Stromkreises, in der ein von der Stromquelle und den Elektroden (70) gebildeter Stromkreis offen ist, und einer Position mit geschlossenem Stromkreis, in der der elektrisch leitende Abschnitt (74) die beiden Elektroden (70) elektrisch verbindet, wobei sich der elektrisch leitende Abschnitt (74) zwischen den beiden Positionen bewegt, wenn ein minimaler Druck auf die Anlagefläche (24) ausgeübt wird.

15. Hilfsvorrichtung (10) nach dem vorhergehenden Anspruch, wobei die Mittel zum Erfassen eines Mindestdrucks (60) ein Vorspannelement (76) aufweist, das zwischen dem elektrisch leitenden Abschnitt (74) und den Elektroden (70) angeordnet ist und dazu neigt, den elektrisch leitenden Abschnitt (74) in eine Position mit offenem Stromkreises vorzuspannen, wobei das Vorspannelement (76) beispielsweise eine Feder oder eine Luftkammer ist, die zwischen dem elektrisch leitenden Abschnitt (74) und den Elektroden (70) angeordnet ist.

16. Kit zur Abgabe eines flüssigen Produkts in ein aufnehmendes Organ Körper eines Patienten, aufweisend eine Vorrichtung (12) zur Abgabe eines flüssigen Produkts und eine Hilfsvorrichtung (10) nach einem der vorangehenden Ansprüche.

## Claims

1. Assistance device (10) for assisting in the use of a device (12) for dispensing a liquid product in the form of drops into a receiving organ of a subject, the dispensing device (12) comprising a tank, the assistance device (10) comprising:
- connecting means (18) for connecting to the dispensing device,
- a bearing surface (24) that bears against the skin of the subject, arranged near a dispensing orifice (14) for dispensing the liquid product,
**characterized in that** the assistance device comprises means for detecting a contact between the bearing surface (24) and the skin of the subject.

2. Assistance device (10) according to the preceding claim, comprising separation means configured to preserve a predetermined distance between the bearing surface (24) and the drop dispensing orifice (14).

3. Assistance device (10) according to any one of the preceding claims, further comprising color detection means (38) for detecting a colour of an area arranged opposite the bearing surface (24) and/or the drop dispensing orifice (14), and possibly an information processing system (36) to compare the colour detected with at least one predetermined colour.

4. Assistance device (10) according to any one of the preceding claims, further comprising locking means for locking the product dispensing adapted to only allow activation of the dispensing device (12) depending on the contact detected and/or a colour detected.

5. Assistance device (10) according to any one of the preceding claims, further comprising optical means (40) intended to be arranged near the drop dispensing orifice (14), and configured to provide information on the dispensing of a drop of the liquid product by the dispensing device (12).

6. Assistance device (10) according to the preceding claim, further comprising inclination measuring means (42) configured to provide information on the inclination of the dispensing device (12) connected to the assistance device (10), and an information processing system (36) on the dispensing of a drop and on the inclination of the dispensing device (12) connected to the assistance device (10) to provide information on the amount of liquid product dispensed.

7. Assistance device (10) according to any one of the preceding claims, comprising weight measuring means (44) for measuring the weight of the dispensing device (12) connected to the assistance device (10), configured to provide information on the amount of liquid product remaining in the dispensing device (12).

8. Assistance device (10) according to any one of the preceding claims, comprising setting means for adjusting the bearing surface (24), so as to be able to vary the distance maintained between the dispensing orifice (14) and at least a part of the bearing surface (24).

9. Assistance device (10) according to any one of the preceding claims, wherein the means for detecting a contact comprise light detection means (30), configured to detect a variation in the light intensity over a part of the bearing surface (24).

10. Assistance device (10) according to any one of the preceding claims, wherein the means for detecting a contact comprise at least one electrode (50, 70) connected to an electrical circuit (52), the electrode (50, 70) being arranged on the bearing surface (24), the electrical circuit (52) being adapted to detect a modification in at least one value chosen from the voltage, current and electrical resistance, when the skin of a subject is in contact with the electrode (50, 70).

11. Assistance device (10) according to any one of the preceding claims, wherein the means for detecting a contact comprise minimum pressure detection means (60) for detecting a minimum pressure exerted on the bearing surface (24).

12. Assistance device (10) according to the preceding claim, wherein the minimum pressure detection means (60) comprise a first air chamber (64) that deforms when the bearing surface (24) is pressed against the skin of the subject, and at least one pressure sensor (62) adapted to be activated when the pressure of the first air chamber (64) reaches a predetermined threshold.

13. Assistance device (10) according to the preceding claim, wherein the minimum pressure detection means (60) comprise a second air chamber (66) of volume less than that of the first air chamber (64), the second air chamber (66) being arranged between the first air chamber (64) and the pressure sensor (62) so that the forces are concentrated on the pressure sensor (62).

14. Assistance device (10) according to any one of claims 11 to 13, wherein the minimum pressure detection means (60) comprise two electrodes (70) connected to a electricity source (72) and an electrically conductive part (74) mounted so as to be able to move between an open circuit position, in which an electric circuit formed by the electricity source and the electrodes (70) is open, and a closed circuit position, in which the electrically conductive part (74) electrically connects the two electrodes (70), the electrically conductive part (74) moving between the two positions when a minimum pressure is exerted on the bearing surface (24).

15. Assistance device (10) according to the preceding claim, wherein the minimum pressure detection means (60) comprise a return element (76) arranged between the electrically conductive part (74) and the electrodes (70) and tending to return the electrically conductive part (74) to the open circuit position, the return element (76) being for example a spring or an air chamber arranged between the electrically conductive part (74) and the electrodes (70).

16. Kit for dispensing a liquid product into a receiving organ of a subject, comprising a device (12) for dispensing liquid product and an assistance device (10) according to any one of the preceding claims.
